# EUROPÄISCHE PATENTSCHRIFT

(11) **EP 2 040 629 B1**
(45) Veröffentlichungstag und Bekanntmachung des Hinweises auf die Patenterteilung: **20.11.2013**
(21) Anmeldenummer: 07785619.3
(22) Anmeldetag: 10.07.2007
(51) Int. Cl.: A61B 17/32, A61B 17/06

(54) **VORRICHTUNG ZUR MINIMAL-INVASIVEN FASZIENENTNAHME**
MINIMALLY INVASIVE FASCIECTOMY DEVICE
DISPOSITIF DE PRÉLÈVEMENT NON INVASIF DE FASCIA

(30) Priorität: 15.07.2006 DE 102006032897
(43) Veröffentlichungstag der Anmeldung: 01.04.2009
(73) Patentinhaber: Universitätsklinikum Schleswig-Holstein, 23538 Lübeck (DE)
(72) Erfinder: HARTMANN, Rolf, 50745 Boras (DE); RUSSLIES, Martin, 23562 Lübeck (DE); ASCHERL, Rudolf, 04319 Leipzig (DE); KÖLLER, Wolfgang, 23566 Lübeck (DE)
(74) Vertreter: Appelt, Christian W.
(86) Internationale Anmeldenummer: PCT/DE2007/001221
(87) Internationale Veröffentlichungsnummer: WO 2008/009265

(56) Entgegenhaltungen:
- WO-A-2004/037070
- US-A1- 2005 085 837
- HARTMANN R ET AL: "ENTWICKLUNG EINES OPERATIONSGERAETES ZUR MINIMAL-INVASIVEN FASCIA LATA-ENTNAHME DESIGNING A SURGICAL DEVICE FOR HARVESTING AUTOLOGOUS FASCIA LATA GRAFTS AS A MINIMAL INVASIVE PROCEDURE" BIOMEDIZINISCHE TECHNIK, FACHVERLAG SCHIELE UND SCHOEN GMBH. BERLIN, DE, Bd. 51, 2006, Seiten 125-130, XP009074905 ISSN: 0013-5585
- GRIEBLING ET AL: "Fascia lata sling cystourethropexy for the manaement of female urinary incontinence" INTERNATIONAL UROGYNECOLOGY JOURNAL, SPRINGER INTERNATIONAL, LONDON, GB, Bd. 9, 1998, Seiten 165-173, XP009093992 ISSN: 0937-3462

## Beschreibung

Die Erfindung betrifft eine Vorrichtung zur minimal-invasiven Faszienentnahme mit nachfolgender Schließung der Öffnung in der Gewebeschicht mit einem chirurgischen Nähfaden.

Die Faszie ist eine wenig dehnbare Hülle einzelner Organe, Muskeln oder Muskelgruppen. Körperfaszien umhüllen die Gesamtmuskulatur des Rumpfes oder die Extremitäten. Bei einer Schwächung des Fasziengewebes kann dieses seine Stützfunktion nicht mehr aufrechterhalten. Es kann dann folgerichtig zur Vorwölbung des zu stützenden Gewebes kommen - bezeichnet als Herniation. Unter bestimmten Umständen ist zur Behebung dieser Funktionseinschränkung ein chirurgischer Eingriff notwendig.

Bekannt sind Verfahren, die die Faszie durch Raffung oder Dopplung wieder spannen bzw. verstärken, sodass sie ihre Haltefunktion zurückgewinnt. Ebenfalls wird Faszienmaterial bei vielen operativen Verfahren als Transplantat (überpflanztes Gewebestück) in unterschiedlicher Form und Größe angewandt. Das Gewebe eignet sich bevorzugt zum Ersatz und zur Wiederherstellung (Rekonstruktion, Augmentation), zur Aufhängung (Suspension), zur Zwischenschaltung (Interposition) und zur Deckung (Okklusion) von Gewebedefekten.

Die genannten Operationsmethoden zur Faszienwiederherstellung bzw. -entnahme sind meist so genannte "offene" Methoden, d. h. man eröffnet die Haut und Unterhaut über dem gesamten Operationsgebiet (Seybold, K.: Die Augmentationsnaht des vorderen Kreuzbandes mit einer Kordel oder einem Fascia lata - Streifen, Universität München, Diss. 1994; Gohrbrandt, E., et al.: Handbuch der Chirurgie, Berlin/de Gruyter, 1965). Dieses beinhaltet eine entsprechende Traumatisierung des umliegenden Gewebes. Zur Verminderung dieser Gewebeschädigung, wie sie bei konventioneller operativer Vorgehensweise entsteht, werden in der Chirurgie minimal-invasive Verfahren entwickelt. Ein Beispiel der Faszienentnahme ist das so genannten Faszienstrippern. Der Anfangsteil des zu entnehmenden Transplantates wird hierbei über einen kleinen Hautschnitt frei präpariert, in den Faszienstripper eingelegt und dieser dann unter der Haut in der gewünschten Länge des Gewebestreifens vorgeschoben. Diese Operationsmethode kann sehr variiert ausgeführt werden, entspricht aber in wesentlichen Ausführungen bekannten Offenbarungen (DE/EP 0 707 456 T1, DE 695 33 893 T 2). Ein am Instrumentengriff zu bedienender Schneidemechanismus trennt die Faszie dann am Stripperanfang unter der Haut.

Ein großer Nachteil dieser minimal-invasiven Methode ist jedoch, dass die so entstandene Faszienlücke nicht wieder verschlossen wird und somit eine Hernie resultieren kann. Neben kosmetischen Beeinträchtigungen können Beschwerden von Seiten der eingeklemmten Muskulatur auftreten.

Für alle nach dem Stand der Technik ausgeführten Operationsmethoden besteht das gleiche Problem: bei einer minimal-invasiven Entnahmemethode mit anschließendem Faszienverschluss besteht die größte Schwierigkeit darin, dass eine Muskelherniation vor der Durchführung der Fasziennaht auftritt oder die Schnittkanten der Faszie wegen hoher Gewebespannung nicht wieder adaptiert werden können. Für das Verschließen der Öffnung sind ebenfalls verschiedenste Vorrichtungen in unterschiedlichster Handhabung bekannt. Am weitesten verbreitet ist die Methode nach der DE 199 44 236 A1; ebenfalls decken die Offenbarungen DE 199 44 236 A 1, DE 200 09 815 U 1 und DE 695 24 130 T 2 einen sehr breiten Funktionsbereich verschiedener Verschlussmethoden ab.

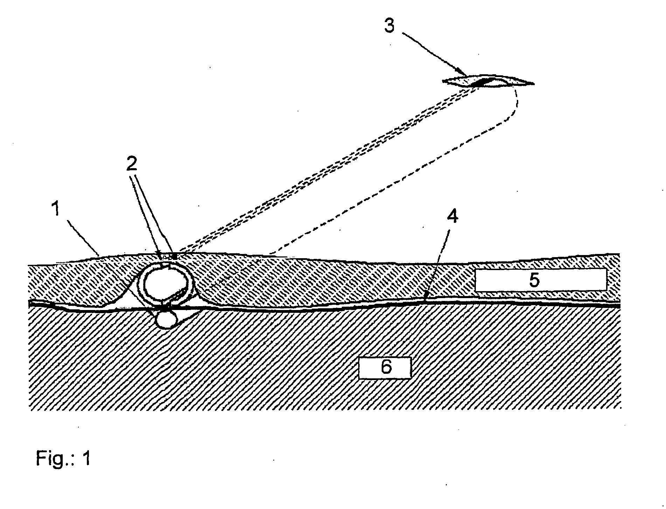

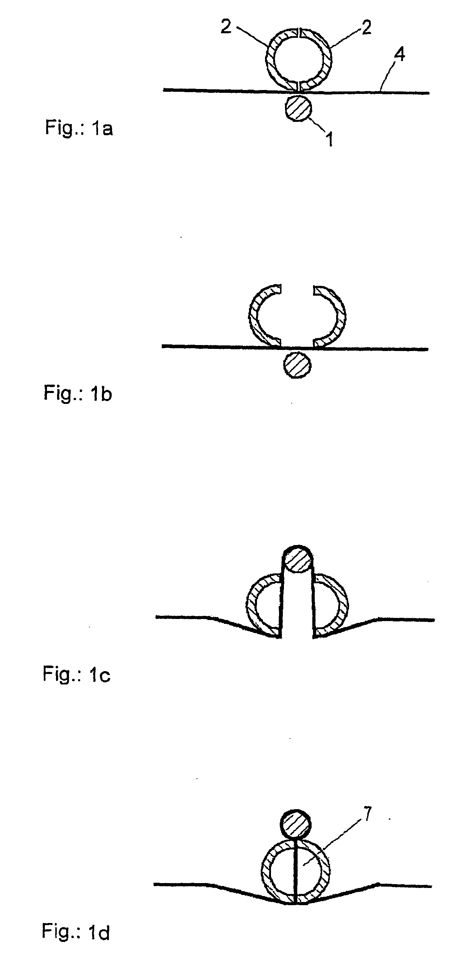

Die WO-A-2004/037070 offenbart eine Vorrichtung zur minimal invasiven Chirurgie, geeignet zur minimal invasiven Faszien-Entnahme, ein rohrförmiges Fixationselement (Fig. 1, No. 35), bestehend aus zwei lösbar miteinander verbundenen Halbrohrschalen (Fig. 1, No. 65 und 69) mit einer an einem proximalen Ende gebildeten keilförmigen Ausnehmung (No. 52) und einem Hebekeil, wobei der Hebekeil unter Auseinanderschieben der lösbar miteinander verbundenen Halbrohrschalen in die keilförmige Ausnehmung einsetzbar ist.

Aufgabe der Erfindung ist es, eine Vorrichtung bereitzustellen, die ein Auseinanderweichen der Faszienschnittkanten verhindert bevor die Fasziennaht durchgeführt wird, um eine minimal-invasive Faszienadaption bzw. -entnahme mit anschließender Fasziennaht zu ermöglichen.

Die Aufgabe wird mit der Vorrichtung nach den Merkmalen des Patentanspruchs 1 gelöst. Vorteilhafte Ausgestaltungen der Erfindung ergeben sich aus den abhängigen Ansprüchen.

Die Erfindung wird im Folgenden unter Bezug auf die beigefügten Zeichnungen erläutert.

Dabei zeigen:
Fig. 1 die Querschnittsansicht des Fixationsprinzips,
Fig. 2 ein halbrohrähnliches Fixationselement,
Fig. 3 den Umlenkstab in Querschnittansicht,
Fig. 4a - 4d die Arbeitsschritte im Arbeitskanal,
Fig. 5 die spiralförmige Kapillare mit Griff.

Vor der Anwendung der erfindungsgemäßen Vorrichtung wird die Fläche des zur Raffung oder Entnahme bestimmten Faszienstückes auf die Haut projiziert. Über den kurzen Seiten des Faszienrechteckes werden kleinstmöglich die Haut und Unterhaut 5 geteilt und die Faszie 4 freigelegt. Danach erfolgt der Schnitt der Faszie 4, den kurzen Seiten des Faszienrechteckes entsprechend. Mit einem spatelartigen Instrument wird nun das Fasziengebiet zwischen den Hautschnitten von der Unterhaut 5 separiert. Ein Umlenkstab 1 wird durch den Faszienschnitt unter die Faszie 4 eingeführt und in Richtung des zweiten Faszienschnittes 3 vorgeschoben. Zwei halbrohrähnliche Fixationseinheiten 2 werden durch den Hautschnitt über der Faszie 4 bzw. unter der Haut eingeführt und in Richtung des zweiten Hautschnittes 3 vorgeschoben. Das Fixationsprinzip wird in der Querschnittsansicht mit dem unter der Faszie 4 eingebrachten Umlenkstab 1 und den beiden unter der Haut und oberhalb der Faszie 4 liegenden halbrohrähnlichen Fixationselementen 2 schematisch dargestellt.

Die Figuren 1a bis 1d zeigen die einzelnen Teilschritte des Entnahmevorgangs. Nach der Separierung der Faszie 4 werden am distalen Hautschnitt die Fixationselemente 2 subkutan entlang des Umlenkstabes 1, der zuvor unter die Faszie 4 eingebracht wurde, eingeführt. In diesem Zustand sind die halbrohrähnlichen Fixationselemente 2 geschlossen und bilden ein rohrförmiges Gerät; der Umlenkstab 1 ist unter die Faszie 4 eingebracht. Der Hauptverschlussapparat 8 wird durch zwei Inbusgewindestifte zusammengehalten. Mittels eines Inbusschlüssels kann der Hauptverschlussapparat 8 geöffnet werden, sodass sich die Fixationselemente 2 parallel voneinander wegbewegen. Nach der Geräteöffnung wird der Umlenkstab 1 zwischen die beiden halbrohrähnlichen Fixationselemente 2 positioniert und auf die am distalen Hautschnitt und dem 2. Hautschnitt 3 eingebrachten Hebekeile 11, 12 aufgesteckt. Nachdem die beiden Fixationselemente 2 auseinander gewichen sind - Fig. 1b - bewegt sich der Umlenkstab 1 relativ zur Faszienebene 4 nach oben und die Fixationselemente 2 nach unten. Wenn das zu raffende bzw. auszuschneidende Gewebestück von dem Umlenkstab 1 aus der Faszienebene 4 herausgezogen wurde, rücken die Fixationselemente 2 wieder aufeinander zu und bringen somit die zukünftigen Schnittkanten zueinander in Kontakt, was in Fig. 1c c schematisch darstellt ist. In Fig. 1d ist schematisch die Schaffung eines Arbeitskanals 7 zur Faszienentnahme abgebildet, indem die zur Fixation benutzten halbrohrähnlichen Fixationselemente 2 so konzipiert sind, dass sie zusammengeführt bei zwischenliegender Faszie 4 ein ungefähr echtes Kreislumen ergeben. Das entstandene Rohrlumen dient nun als Arbeitskanal 7. Da die anderen Weichteile außerhalb des Rohrlumens liegen, können die weiteren Arbeitsschritte ohne visuelle Überwachung erfolgen.

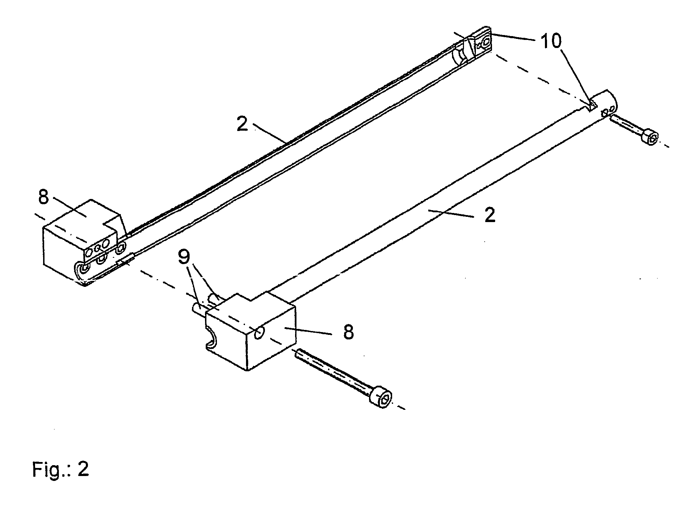

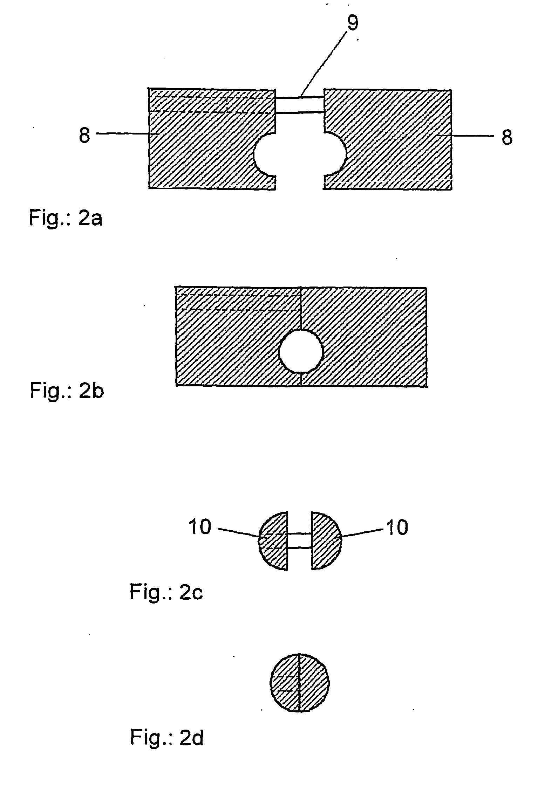

Um die Funktion des Fixationssystems zu gewährleisten, müssen die halbrohrähnlichen Fixationselemente 2 parallel zueinander bewegt werden. Dies wird durch eine Verschlussvorrichtung 10 an beiden Enden des Gerätes ermöglicht, welche die halbrohrähnlichen Fixationselemente 2 zu einer Geräteeinheit als Hauptverschlussapparat 8 verbindet. Da die Schneidkanten der Fixationselemente 2 präzise aufeinander treffen müssen, ist der Hauptverschlussapparat 8 mit Gleitstiften 9 als Führungselemente ausgestattet. Das Verschlussprinzip der jeweiligen Verschlussvorrichtungen an den Geräteenden wird in Querschnittsansichten in den Figuren 2a bis 2d veranschaulicht. Wie in Fig. 2 und in den Figuren 2a bis 2d dargestellt, sind die Verschlussvorrichtungen unterschiedlich dimensioniert. Das Geräteende, welches als erstes über den 1. Hautschnitt unter die Faszie eingeführt wird, beinhaltet eine klein dimensionierte Verschlusseinheit 10, die über den 2. Hautschnitt zugänglich ist. Am anderen Geräteende befindet sich der Hauptverschlussapparat 8, welcher durch zwei Gleitstifte 9 geführt und mit Hilfe eines Gewindestabes bewegt wird. Wegen seiner Größe bleibt dieser Teil des Gerätes außerhalb der Haut platziert. Er dient gleichzeitig als Gerätegriff. Die Querschnitte in den Figuren 1a bis 1c zeigen die Einheiten im geöffneten und geschlossenen Zustand.

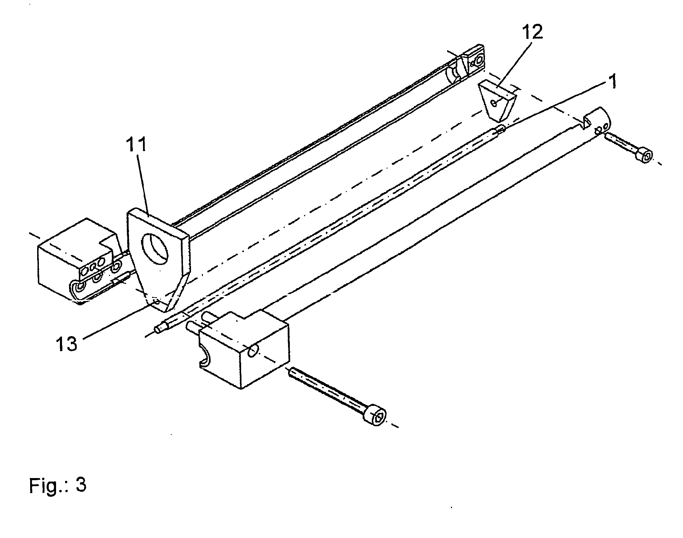

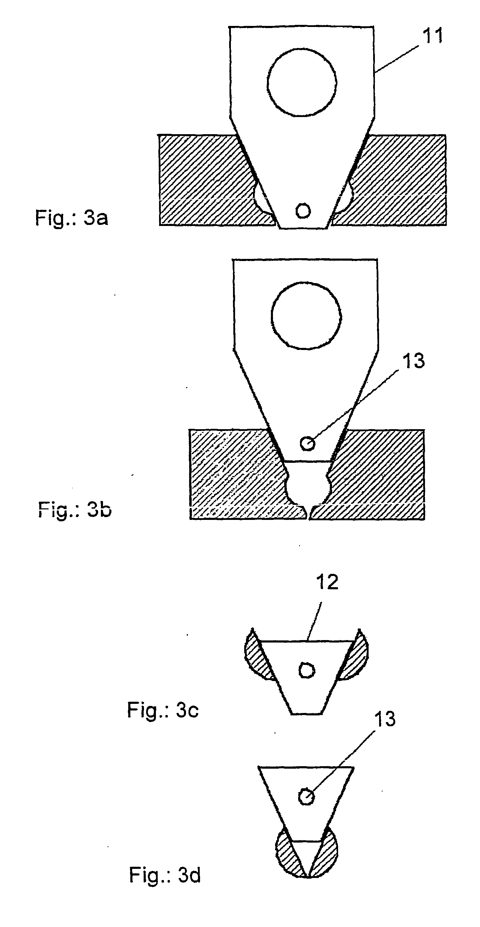

Das Hebeprinzip des Umlenkstabes 1 funktioniert mittels schiefer Ebenen. Diese wandeln die Verschlussbewegung, also das Aufeinanderzugleiten der halbrohrähnlichen Fixationselemente 2, in eine Hebebewegung um. Auf die gefrästen Enden des Umlenkstabes 1 werden keilartige Aufsätze als Hebekeile 11, 12 gesteckt und in die Hebeschächte, welche an beiden Enden des Gerätes, wie in Fig. 3 dargestellt, liegen, platziert. Beim Geräteverschluss gleiten die keilförmigen Aufsätze 11, 12 in den Schächten nach oben. Mit ihnen wird der Umlenkstab 1 mit der Faszie 4 nach oben durchgehoben. Das Hebeprinzip des Umlenkstabes 1 wird in einer Querschnittansicht der jeweiligen Keilaufsätze 11, 12 in den Hebeschächten in den Teilfiguren 3a bis 3d sichtbar. Beim Schließen der Verschlusseinheiten werden die Keilaufsätze 11, 12 nach oben gedrückt. Der in den kleinen Bohrungen 13 gehaltene Umlenkstab wird nach oben zwischen den halbrohrähnlichen Fixationseinheiten 2 durchgehoben. Die dargestellten Querschnitte 3a, 3b und 3c, 3d zeigen die Einheiten im geöffneten und geschlossenen Zustand.

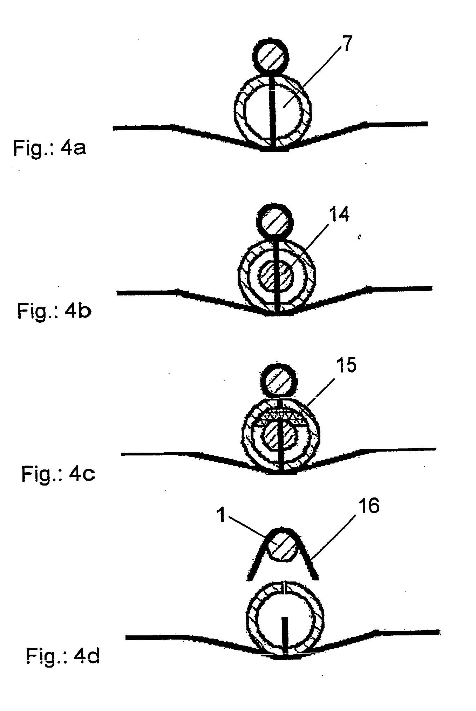

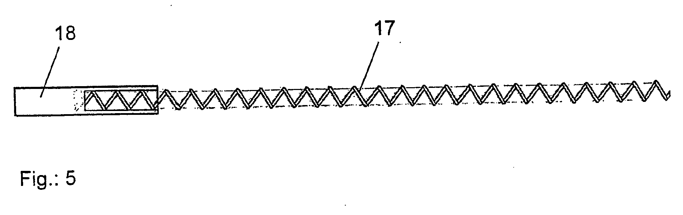

Der weitere Arbeitsablauf zur Bearbeitung der Faszie 4 wird in den Figuren 4a bis 4d schematisch dargestellt und erfolgt im Arbeitskanal 7 - entsprechend Fig. 4a. Zur weiteren Schienung wird eine gabelähnliche Konstruktion 14 mit jeweils einem links und rechts der Faszie 4 liegenden Gabelschenkel zentrisch in den Arbeitskanal 7 eingebracht - dargestellt in Fig. 4b. Als weiterer Arbeitsschritt wird ein Skalpellschlitten 15 oberhalb dieser Schiene in den Arbeitskanal 7 eingeführt, was Fig. 4c zeigt. Beim Vorschub des Skalpells auf der Gabel wird die Faszie im oberen Bereich des Arbeitskanals 7 durchtrennt und das Stück, welches um den Umlenkstab 1 verläuft, wird als Faszienstreifen 16 frei - schematisiert in Fig. 4d. Der Faszienstreifen 16 kann nun zusammen mit dem Umlenkstab 1 entnommen werden. Die Naht der immer noch adaptierten Faszie 4 erfolgt über eine spiralförmige Kapillare 17 nach Fig. 5. Die Kapillare 17 wird auf der Schienung 14 vollständig bis zum anderen Geräteende vorgedreht. Im Lumen der Kapillare wird ein Faszienfaden eingespült und am anderen Ende ergriffen. Durch Zurückdrehen der Spirale mittels eines Griffs 18 verbleibt der Faszienfaden als fortlaufende, überwendelnde Naht in der Faszie 4. Abschließend werden die Nahtüberstände mit der intakten Faszie 4 vernäht, wonach der weitere Wundverschluss erfolgt.

Die erfindungsgemäße Vorrichtung eignet sich damit sowohl für eine Raffungsoperation als auch für eine Transplantatentnahme.

### Bezugszeicheziliste:

- 1:: Umlenkstab
- 2:: Fixationselemente
- 3:: 2. Hautschnitt
- 4:: Faszie
- 5:: Haut und Unterhaut
- 6:: Muskel
- 7:: Arbeitskanal
- 8:: Hauptverschlussapparat
- 9:: Gleitstift
- 10:: Verschlusssystem
- 11:: großer Hebekeil
- 12:: kleiner Hebekeil
- 13:: Aufnahmebohrung
- 14:: Gabelschiene
- 15:: Skalpellschlitten
- 16:: Faszienstreifen
- 17:: Kapillare
- 18:: Kapillargriff

## Patentansprüche

1. Vorrichtung zur minimal-invasiven Faszienentnahme,
aufweisend:
ein rohrförmiges Fixationselement, bestehend aus zwei lösbar miteinander verbundenen Halbrohrschalen (2, 2) mit jeweils einer an einem proximalen und an einem distalen Ende gebildeten keilförmigen Ausnehmung,
einen Umlenkstab (1), dessen Länge im Wesentlichen dem Abschnitt zwischen den Ausnehmungen entspricht, und
Hebekeile (11, 12) mit jeweils an ihrem unteren Abschnitt angeordneten Aufnahmen (13) zum lösbaren Verbinden der Hebekeile (11, 12) mit dem Umlenkstab (1),
wobei die Hebekeile (11, 12) unter Auseinanderschieben der lösbar miteinander verbundenen Halbrohrschalen (2, 2) in die keilförmigen Ausnehmungen einsetzbar sind.

2. Vorrichtung nach Anspruch 1, wobei die Hebekeile (11, 12) unterschiedliche Abmessungen aufweisen.

3. Vorrichtung nach einem der vorhergehenden Ansprüche, wobei die Halbrohrschalen (2, 2) mittels eines Gewindestifts lösbar miteinander verbunden sind.

4. Vorrichtung nach einem der vorhergehenden Ansprüche, wobei die miteinander verbundenen Halbrohrschalen (2, 2) einen Arbeitskanal (7) bilden.

5. Vorrichtung nach Anspruch 4, wobei der Arbeitskanals (7) zur Aufnahme einer Gabelschiene (14) eingerichtet ist.

6. Vorrichtung nach einem der Ansprüche 4 und 5, wobei der Arbeitskanal (7) zur Aufnahme einer einen Faszienfaden aufnehmenden, spiralförmigen Kapillare (17) eingerichtet ist.

## Claims

1. An apparatus for minimal invasive fasciectomy, comprising:
a tubular fixation element comprising two semitubular shells (2, 2) which are detachably connected to each other, each having a wedge-shaped recess formed at a proximal and a distal end thereof;
a deflecting bar (1), the length of which substantially corresponds to the portion between the recesses; and
lifting wedges (11, 12) each having receptacles (13) arranged at the lower portion thereof, for detachably connecting the lifting wedges (11, 12) to the deflecting bar (1),
wherein the lifting wedges (11, 12) can be inserted into the wedge-shaped recesses by sliding apart the semitubular shells (2, 2) detachably connected to each other.

2. The apparatus according to claim 1, wherein the lifting wedges (11, 12) have different dimensions.

3. The apparatus according to any one of the preceding claims, wherein the semitubular shells (2, 2) are detachably connected to each other by means of a setscrew.

4. The apparatus according to any one of the preceding claims, wherein the semitubular shells (2, 2) connected to each other form a working channel (7).

5. The apparatus according to claim 4, wherein the working channel (7) is adapted for receiving a fork-shaped rail (14).

6. The apparatus according to any one of claims 4 and 5, wherein the working channel (7) is adapted for receiving a helical capillary (17) receiving a fascial fibre.

## Revendications

1. Dispositif pour une fasciectomie peu invasive, comportant :
- un élément de fixation tubulaire, formé par deux demi-coques (2, 2) semi-tubulaires assemblées l'une à l'autre de manière amovible et comportant chacune un évidement cunéiforme, formé au niveau d'une extrémité proximale et au niveau d'une extrémité distale,
- une tige de déviation (1) dont la longueur correspond sensiblement à la partie comprise entre les évidements, et
- des cales de levage (11, 12) comportant chacune des logements (13) disposés au niveau de leur partie inférieure, pour permettre un assemblage amovible entre les cales de levage (11, 12) et la tige de déviation (1),
dans lequel dispositif lesdites cales de levage (11, 12) peuvent être insérées dans les évidements cunéiformes après avoir écarté l'une de l'autre les demi-coques (2, 2) semi-tubulaires assemblées l'une à l'autre de manière amovible.

2. Dispositif selon la revendication 1, dans lequel les cales de levage (11, 12) ont des dimensions différentes.

3. Dispositif selon l'une quelconque des revendications précédentes, dans lequel les demi-coques (2, 2) semi-tubulaires sont assemblées l'une à l'autre de manière amovible au moyen d'une tige filetée.

4. Dispositif selon l'une quelconque des revendications précédentes, dans lequel les demi-coques (2, 2) semi-tubulaires assemblées l'une à l'autre forment un conduit de travail (7).

5. Dispositif selon la revendication 4, dans lequel le conduit de travail (7) est configuré pour recevoir un rail en Y (14).

6. Dispositif selon les revendications 4 et 5, dans lequel le conduit de travail (7) est configuré pour recevoir un capillaire (17) en forme de spirale recevant un fil de fascia.
